# EUROPEAN PATENT SPECIFICATION

(11) **EP 2 336 776 B1**
(45) Date of publication and mention of the grant of the patent: **05.03.2014**
(21) Application number: 08800549.1
(22) Date of filing: 29.08.2008
(51) Int. Cl.: G01N 33/543, G01N 33/558

(54) **LIQUID TEST STRIP**
FLÜSSIGKEITSTESTSTREIFEN
BANDELETTE DE TEST POUR LIQUIDE

(43) Date of publication of application: 22.06.2011
(73) Proprietor: Actherm Inc., Hsinchu 30078 (TW)
(72) Inventor: HSIEH, Chih-Wei, Hsinchu Country 302 Taiwan (CN); HSIEH, Wen-Pin, Miaoli Country 352 Taiwan (CN); WU, Yi-Jen, Taiwan (CN)
(74) Representative: Lang, Christian
(86) International application number: PCT/CN2008/001551
(87) International publication number: WO 2010/022543

(56) References cited:
- EP-A2- 0 366 241
- WO-A1-00/42434
- WO-A1-2004/086042
- WO-A2-2004/018623
- CN-A- 1 854 728
- CN-A- 1 954 214
- US-A1- 2004 082 077
- US-A1- 2008 139 405
- US-B1- 6 656 745
- AHMAD ET AL: "Effects of membrane cast thickness on controlling the macrovoid structure in lateral flow nitrocellulose membrane and determination of its characteristics", SCRIPTA MATERIALIA, ELSEVIER, AMSTERDAM, NL, vol. 57, no. 8, 28 July 2007 (2007-07-28), pages 743-746, XP022176499, ISSN: 1359-6462, DOI: DOI:10.1016/J.SCRIPTAMAT.2007.06.037

## Description

### BACKGROUND OF THE INVENTION

### 1. Technical Field

The present invention relates to analytical strips, and more particularly to an analytical strip for biochemical or immunological assays.

### 2. Description of Related Art

Conventional analytical strips used in biochemical or immunological assays usually have a substrate or a baseboard provided with a channel or a microfluidic channel. E.g. WO 2004/086042 Al discloses a multiple-channel test device, which uses a laser etching method to provide the multiple-channels for testing the analytes. While such channel is typically bordered by a non-absorptive material, and the viscosity of the fluid sample to be analyzed is usually high for the sample is mainly composed of proteins or carbohydrates, part of the fluid sample tends to adhere to the surface of the channel and will not be reacted. Such scenario, if happens, will not only disadvantageously cause the waste of the fluid sample to be analyzed, but also will adversely affects the accuracy of quantifying assays.

In addition, the conventional analytical strip may facilitate the flow of the fluid sample by microfluidic channels so that the fluid sample will be delivered via the capillary force exerted by the structures of such channels to the reaction area. For example, US 2004/082077 A1 discloses a diagnostic device making use of the capillary force of the channels to drive the fluid samples flow. US 6,656,745 B1 also utilizes capillary channels for transporting the fluid samples. Another alternative approach to deliver the fluid sample involves applying a driving force, such as by a pressurizing means, at the time the fluid sample is introduced into the channel so that the fluid sample is propelled to the reaction area through the channel. However, either one of the aforementioned approaches tends to cause air bubbles occurring after the fluid sample is introduced into the channel. These bubbles, either large or small, will block the channel and result in inaccurate analyzing results.

Furthermore, the manufacturing process of the channels or microfluidic channels on the current substrates is usually involves molding, injection forming or imprinting. Consequently, the analytical strips comprising those abovementioned substrates have to be made of high-priced micro-injection molds manufactured by using micro-machining or LIGA (abbreviation of "Lithographie GalVanoformung Abformung", or "Lithography Electroforming Micro Molding" in english) technique. The micro-injection mold used in the manufacturing process tends to wear out rapidly, which results in the relatively high cost.

### SUMMARY OF THE INVENTION

In an attempt to overcome the recited drawbacks and shortcomings of the conventional analytical strips, the present invention provides an analytical strip that comprises a substrate having a channel provided concavely on an upper surface of the substrate.

Another object of the present invention is to provide an analytical strip that has absorptive nitrocellulose layers and has a constant volumetric absorptive capacity, thus the analytical strip allows a quantitative assay to be conducted via controlling the volume of the nitrocellulose layers.

Still another object of the present invention is to provide an analytical strip that has absorptive nitrocellulose layers with a hollow-matrix configuration, which is capable of destroying the air bubbles in the fluid sample when the fluid sample flows through the hollow matrix, as well as preventing the bubbles from blocking the channel or the microfluidic channel of the substrate. Thus, an accurate result of the quantitative assay could be assured.

The above-mentioned objects are solved by the analytical strip according to claim 1. Advantageous improvements are described by dependant claims.

In order to accomplish the objects mentioned above, the technical solutions provided by the present invention are as below:

An analytical strip comprises a substrate having a channel provided concavely on an upper surface. The channel comprises a first area for receiving a fluid sample, a second area for delivering the fluid sample, and a third area where the fluid sample reacts. These three areas are connected sequentially. The analytical strip is characterized in that nitrocellulose layers are formed at each bottom of both the second and the third area, and the conformation of the nitrocellulose layers is a hollow matrix. In addition, the nitrocellulose layer of the second area has an average thickness that is smaller than the thickness of the nitrocellulose layer of the third area. The analytical strip also comprises a reaction material formed in the hollow matrices of the nitrocellulose layers.

The advantages of the analytical strip of this invention are as below:
Because the analytical strip has a thin absorptive nitrocellulose layer on the bottom of channel and the thin absorptive nitrocellulose layers act as sample delivering and/or separating function, the channel thus has lower residual of samples in contrast to the traditional microfluidic channel, and low volume of samples needed for multi-analytes detection in a test is realized. Because the absorptive nitrocellulose layers and has a constant volumetric absorptive capacity, thus the analytical strip allows a quantitative assay to be conducted via controlling the volume of the nitrocellulose layers. Because the absorptive nitrocellulose layers with a hollow-matrix configuration, which is capable of destroying the air bubbles in the fluid sample when the fluid sample flows through the hollow matrix, as well as preventing the bubbles from blocking the channel or the microfluidic channel of the substrate, an accurate result of the quantitative assay could be assured.

### BRIEF DESCRIPTION OF THE DRAWINGS

The invention as well as a preferred mode of use, further objects and advantages thereof will be best understood by reference to the following detailed description of illustrative embodiments when read in conjunction with the accompanying drawings, wherein:
Fig. 1A is a perspective view of an analytical strip according to a first embodiment of the present invention;
Fig. 1B is a cross-sectional view of the analytical strip according to the first embodiment of the present invention;
Fig. 2A is a perspective view of an analytical strip disclosed herein; and
Fig. 2B is a cross-sectional view of the analytical strip disclosed herein.

### DETAILED DESCRIPTION OF THE PREFERRED EMBODIMENTS

While the present invention proposes an analytical strip, the physical and chemical principles, as well as solution applying techniques it employs are known to one skilled in the art and need not be discussed at any length herein. Meanwhile, the accompanying drawings referred to in the following description are provided for illustrative purposes and need not to be made to scale.

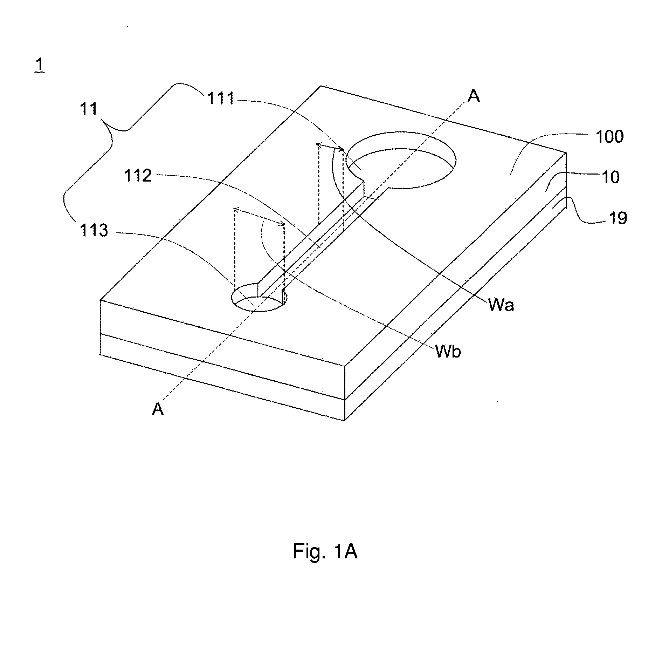

Fig. 1A is a perspective view of an analytical strip according to a first embodiment of the present invention. The analytical strip 1 comprises a substrate 10 and a backing plate 19. The substrate 10 has an upper surface 100 concavely provided with a channel 11. The channel 11 includes a first area 111, a second area 112 and a third area 113 that are connected sequentially. The first area 111 is to receive a fluid sample to be analyzed. The fluid sample is introduced to the first area 111 and then delivered by the second area 112 to the third area 113 where analytes of the fluid sample react and are then detected. In a preferred mode of the present invention, the substrate 10 is made of a biocompatible material.

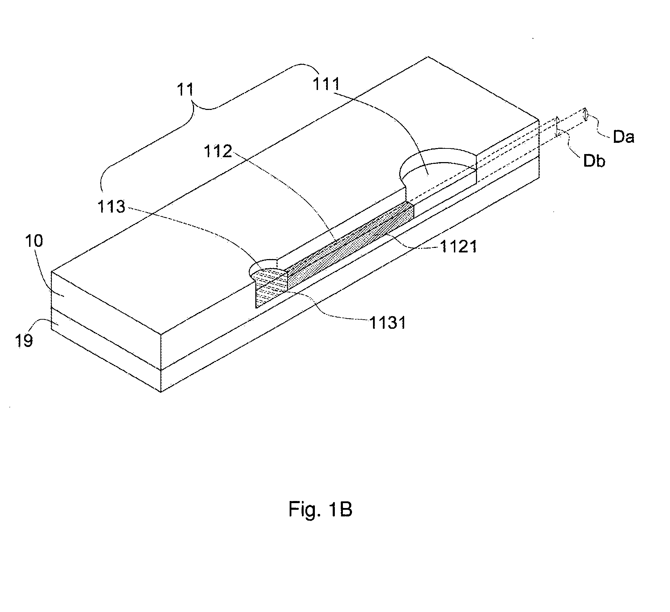

Now please refer to Fig. 1B, which is a cross-sectional view of the analytical strip 1 taken along Line A-A of Fig. 1A. As shown in Fig. 1B, the nitrocellulose layers 1121 and 1131 are formed respectively at both bottoms of the second area 112 and the third area 113. Each of the nitrocellulose layers 1121 and 1131 has a hollow-matrix conformation that contains a reaction material therein. The composition of the reaction material varies from the category of the analyte in the fluid sample to be detected. The porous hollow matrix serves to absorb the fluid sample coming from the first area 111 and the analytes in the fluid sample can react with the pre-embedded reagents in the nitrocellulose layer 1131. Since the nitrocellulose layers 1121 and 1131 are absorptive, the channel 11 thus has lower residual of samples in contrast to the traditional microfluidic channel, and low volume of samples needed for multi-analytes detection in a test is realized. In addition, when the fluid sample flows through the nitrocellulose layers 1121 and 1131, the hollow-matrix conformation will destroy the air bubbles in the fluid sample, thereby preventing the bubbles from blocking the channel 11.

The analytical strip 1 of the present invention can be applied to either biochemical assays or immunological assay. To detect different analytes of the physiological fluid needs different assays, and different categories of assays require different kinds of reaction materials, which result in different categories of signals. A biochemical quantitative assay, for example, is usually carried out via the enzymatic reaction of the analytes in the biological fluid sample and a chemical luminating reagent, which is catalyzed by the suitable enzymes, to generate optical signals with specific wavelengths for detection. Accordingly, the reaction materials of the analytical strip 1, when applied to the biochemical quantitative assay, will mainly comprise enzymes and the corresponding chemical reagents. On the other hand, when the scenario comes to the quantitative detection of a certain protein in the physiological fluid sample, such as a-fetoprotein, the analytical assay usually utilize a antibody which can specifically recognize the targeted protein and other corresponding chemical reagents to generate detectable signals. Accordingly, the reaction materials of the analytical strip 1, when applied to the quantitative immunoassay, will mainly comprise antibodies and the corresponding reagents. Therefore, the analytical strip 1 of the present invention is adaptive to quantitative detection of various analytes in different types of physiological fluidic specimens (e.g., urine and blood).

Please refer to Fig. 1B, the nitrocellulose layer 1121 of the second area 112 has an average thickness Da which is not greater than the average thickness Db of the nitrocellulose layer 1131 of the third area 113, namely that Da is smaller than Db. Furthermore, in order to reduce the volume of the fluid sample required, the width Wa of the second area 112 and the width Wb of the third area 113 (shown in Fig. 1A) are both preferably 0.3 mm at least.

The nitrocellulose layers 1121 and 1131 are formed as the following steps. Firstly, a nitrocellulose powder is mixed with an organic solvent containing esters and ketones to form a nitrocellulose solution. The nitrocellulose solution is then applied to the bottoms of the second and third areas 112 and 113 via a casting process. After drying, the nitrocellulose layers 1121 and 1131 are formed respectively at the bottoms of the second areas 112 and third area 113. For a better result of the casting process, the surface roughness (Ra) of the channel 11 preferably ranges from about 3µm to about 50µm.

As previously described, after drying the nitrocellulose solution applied onto the bottoms of the second areas 112 and third area 113 forms the nitrocellulose layers 1121 and 1131 which both have the hollow-matrix conformation. In order to obtain a hollow matrix with a better structure, the nitrocellulose powder preferably has a volume that is about nine times to the volume of the organic solvent containing esters and ketones. Because each volumetric unit of nitrocellulose has a constant absorptive capacity, the required volume of the nitrocellulose solution can be derived from the desired volume of the fluid sample to be adsorbed and analyzed before casting. As a result, the required volume of the fluid sample of the analytical strip 1 will be fixedly set, so that the resultant analytical strip 1 is suitable for an assay in a small volume.

Described herein is that after the nitrocellulose layers 1121 and 1131 are solidified at the bottom of the second and third areas 112 and 113, a reaction solution containing reaction materials is injected into the nitrocellulose layers 1121 and 1131, followed by another drying process, such as air-drying or lyophilization. The reaction materials dried in the nitrocellulose layers 1121 and 1131 will be in the form of powder.

As described previously, the reaction materials can be added after the solidification of the nitrocellulose layers 1121 and 1131 and then dried therein. In the present invention, the nitrocellulose layers 1121 and 1131 and the reaction materials are formed simultaneously. The nitrocellulose solution that contains the nitrocellulose powder and the organic nitrocellulose solution composed of esters and ketones can mix with the reaction solution having the reaction material in advance, before casting onto the bottoms of the second and third areas 112 and 113. After the drying process (ie, air-drying or lyophilization), the nitrocellulose layers 1121 and 1131 are solidified while the reaction materials are left therein in the form of powder.

The above-described first embodiment of the present invention is an analytical strip having a substrate comprising a channel having three areas. Based on the concept of the present invention, the channel may further comprise a fourth area for accommodating excessive fluid sample introduced into the channel. A second embodiment of the present invention given below is an analytical strip that has a channel including four areas.

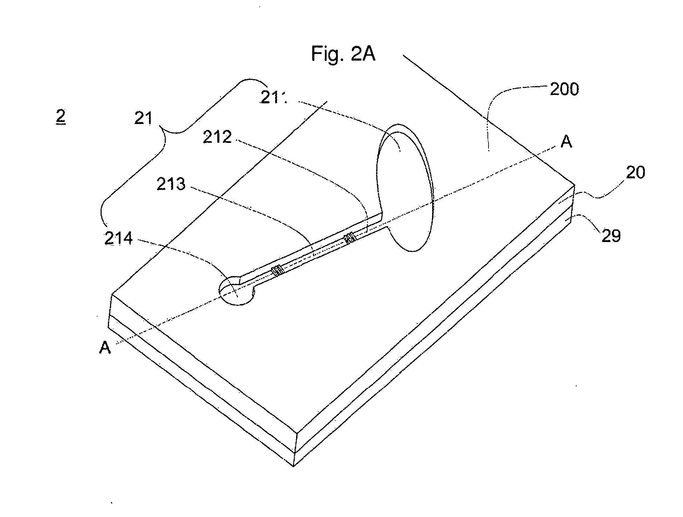

Fig. 2A is a perspective view of the analytical strip according to the second embodiment of the present invention. A substrate 20 of the analytical strip 2 has an upper surface 200 concavely provided with a channel 21. The channel 21 includes a first area 211 for receiving a fluid sample to be analyzed, a second area 212 for delivering the fluid sample, a third area 213 and a fourth area 214. These four areas 211, 212, 213 and 214 are connected sequentially. The fluid sample is introduced to the first area 211 and then delivered via the second area 212 to the third area 213 where the analytes of the fluid sample are reacted.

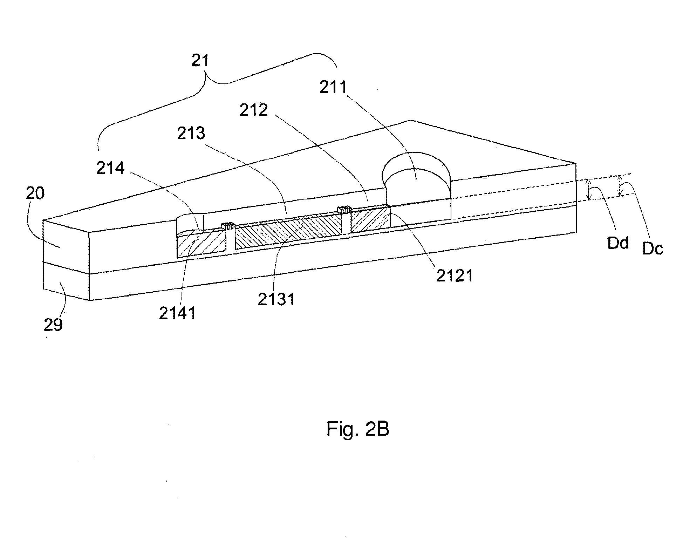

Please refer to Fig. 2B, which is a cross-sectional view of the analytical strip 2 taken along Line A-A of Fig. 2A. Nitrocellulose layers 2121 and 2131 are formed at bottoms of the second area 212 and the third area 213 respectively. In addition, the nitrocellulose layer 2121 of the second area 212 has an average thickness Dc that equals to the average thickness Dd of the nitrocellulose layer 2131 of the third area 213 (for comparison reasons only). Similar to the second and third areas 212 and 213, a nitrocellulose layer 2141 is also formed at the bottom of the fourth area 214 and also has a hollow-matrix conformation for accommodating the excess fluid sample. The nitrocellulose layer 2141 at the bottom of the fourth area 214 is made in the same way as the nitrocellulose layers 2121 and 2131. Namely, the nitrocellulose layers 2121, 2131 and 2141 are formed by casting a nitrocellulose solution onto the bottoms of the second, third and fourth areas 212, 213, and 214, followed by a drying process.

Moreover, The reaction materials can either be formed after the solidification of the nitrocellulose layers 2121, 2131 and 2141, or, alternatively, be formed simultaneously with the nitrocellulose layers 2121, 2131 and 2141 via the similar processes described in the first embodiment. The reaction materials in the nitrocellulose layers 2121, 2131 and 2141 are also in the form of powder.

In addition, in the second embodiment, the structure, dimensions and connective relationships of the first, second and third areas, the preferred material of the substrate, the surface roughness of the channel, the conformation and forming method of the nitrocellulose layers, the preferred ingredients of the nitrocellulose solution and the preferred ratio therebetween, and the preferred composition of the reaction material are all similar to those described in the first embodiment of the present invention and need not to be described herein in further detail.

The present invention has been described with reference to the preferred embodiments and it is understood that the embodiments are not intended to limit the scope of the present invention.

## Claims

1. An analytical strip primarily comprising a substrate having a channel provided concavely on an upper surface thereof, wherein said channel comprises a first area for receiving a fluid sample to be analyzed, a second area and a third area and these three areas are connected sequentially, the analytical strip being **characterized in that**:
each of the second and the third area of the channel has a bottom provided with a nitrocellulose layer, having a hollow-matrix conformation, wherein a reaction material is formed in the hollow-matrix conformation of each of the nitrocellulose layers, wherein the reaction material in the hollow-matrix conformation is in a powder form and formed by mixing a reaction solution containing the reaction material with the nitrocellulose solution and then casting onto the bottoms of the second and third areas, followed by a drying process, so that the nitrocellulose solution forms the nitrocellulose layers, while the reaction material is left in the nitrocellulose layers in the form of powder; and wherein the second area is configured for delivering the fluid sample by absorbing the fluid sample coming from the first area and delivering the fluid sample to the third area and the third area is configured for reacting and detecting analytes of the fluid sample, wherein each of the second area and the third area has a width of at least 0.3 mm, and the nitrocellulose layer of the third area is capable of absorbing a constant volume of the fluid sample coming from the second area, the nitrocellulose layer of the second area having an average thickness, which is smaller than an average thickness of the nitrocellulose layer of the third area.

2. The analytical strip of Claim 1, wherein the nitrocellulose solution is a mixture of nitrocellulose powder and an organic solvent containing esters and ketones.

3. The analytical strip of Claim 2, wherein the nitrocellulose powder is mixed with the solvent containing esters and ketones at a volumetric ratio of 1: 9.

4. The analytical strip of Claim 1, wherein the substrate is made of a biocompatible material.

5. The analytic strip of Claim 1, wherein the channel further comprises a fourth area having a bottom provided with a nitrocellulose layer having a hollow-matrix conformation for accommodating excess of the fluid sample.

6. The analytical strip of Claim 5, wherein the reaction material is formed in the hollow-matrix conformation of the nitrocellulose layer of the fourth area, wherein the reaction material in the hollow-matrix conformation is in a powder form and is formed by mixing a reaction solution containing the reaction material with the nitrocellulose solution and then casting onto the bottoms of the second area, the third area and the fourth area followed by a drying process, so that, so that the nitrocellulose solution forms the nitrocellulose layers, while the reaction material is left in the nitrocellulose layers in the form of powder.

## Patentansprüche

1. Analysestreifen, der hauptsächlich ein Substrat umfasst, das einen Kanal aufweist, der konkav an dessen oberen Oberfläche bereitgestellt ist, wobei der Kanal einen ersten Bereich zur Aufnahme einer zu analysierenden Flüssigkeitsprobe, einen zweiten Bereich und einen dritten Bereich umfasst, wobei diese drei Bereiche in Reihe verbunden sind, wobei der Analysestreifen **dadurch gekennzeichnet ist, dass**:
sowohl der zweite als auch der dritte Bereich des Kanals einen Boden aufweisen, der mit einer Nitrozelluloseschicht versehen ist, die eine Hohlmatrixkonformation aufweist, wobei ein Reaktionsmaterial in der Hohlmatrixkonformation von jeder der Nitrozelluloseschichten ausgebildet ist, wobei das Reaktionsmaterial in der Hohlmatrixkonformation in einer Pulverform ausgebildet ist und durch Mischen einer ein erstes Reaktionsmaterial enthaltenden Reaktionslösung mit der Nitrozelluloselösung und anschließende Gießen auf den Boden des zweiten und des dritten Bereichs ausgebildet ist, gefolgt von einem Trocknungsprozess, so dass die Nitrozelluloselösung die Nitrozelluloseschichten ausbildet, während das Reaktionsmaterial in den Zelluloseschichten in der Form von Pulver verbleibt, wobei der zweite Bereich auf das Bereitstellen der Flüssigkeitsprobe durch Absorbieren der aus dem ersten Bereich kommenden Flüssigkeitsprobe und Bereitstellen der Flüssigkeitsprobe für den dritten Bereich ausgelegt ist und wobei der dritte Bereich zum Reagieren und Detektieren von Analyten der Flüssigkeitsprobe ausgelegt ist, wobei sowohl der zweite Bereich als auch der dritte Bereich eine Breite von mindestens 0,3 mm aufweisen, wobei die Nitrozelluloseschicht des dritten Bereichs in der Lage ist, ein konstantes Volumen der aus dem zweiten Bereich kommenden Flüssigkeitsprobe zu absorbieren, und wobei die Nitrozelluloseschicht des zweiten Bereichs eine durchschnittliche Dicke aufweist, die kleiner ist als eine durchschnittliche Dicke der Nitrozelluloseschicht des dritten Bereichs.

2. Analysestreifen nach Anspruch 1, wobei die Nitrozelluloselösung eine Mischung aus Nitrozellulosepulver und einem organischen Lösungsmittel ist, das Ester und Ketone enthält.

3. Analysestreifen nach Anspruch 2, wobei das Nitrozellulosepulver mit dem Lösungsmittel, das Ester und Ketone enthält, mit einem Volumenverhältnis von 1:9 gemischt wird.

4. Analysestreifen nach Anspruch 1, wobei das Substrat aus einem biokompatiblen Material hergestellt ist.

5. Analysestreifen nach Anspruch 1, wobei der Kanal ferner einen vierten Bereich umfasst, der einen Boden aufweist, der mit einer Nitrozelluloseschicht versehen ist, die eine Hohlmatrixkonformation aufweist, um überschüssige Flüssigkeit der Flüssigkeitsprobe aufzunehmen.

6. Analysestreifen nach Anspruch 5, wobei das Reaktionsmaterial in der Hohlmatrixkonformation der Nitrozelluloseschicht des dritten Bereichs ausgebildet ist, wobei das Reaktionsmaterial in der Hohlmatrixkonformation in einer Pulverform ist und durch Mischen einer Reaktionslosung, die das Reaktionsmaterial enthält, und der Nitrozelluloselösung und anschließendem Gießen auf den Boden des zweiten Bereichs, des dritten Bereichs und des vierten Bereichs gefolgt von einem Trocknungsprozess gebildet ist, so dass die Nitrozelluloselösung die Nitrozelluloseschichten ausbildet, während das Reaktionsmaterial in den Nitrozelluloseschichten in der Form von Pulver verbleibt.

## Revendications

1. Bandelette analytique comprenant principalement un substrat ayant un canal prévu de manière concave sur une surface supérieure de celui-ci, ledit canal comprenant une première zone pour recevoir un échantillon de fluide à analyser, une seconde zone et une troisième zone et ces trois zones étant reliées de manière séquentielle, la bandelette analytique étant **caractérisée en ce que** chaque zone, la seconde et la troisième zone du canal a un fond équipé d'une couche de nitrocellulose ayant une conformation de matrice creuse, un matériau de réaction étant formé dans la conformation de matrice creuse de chacune des couches de nitrocellulose, le matériau de réaction dans la conformation de matrice creuse étant sous forme de poudre et étant formé en mélangeant une solution de réaction contenant le matériau de réaction avec la solution de nitrocellulose et en moulant ensuite sur les fonds de la seconde et de la troisième zone, ceci étant suivi par un processus de séchage si bien que la solution de nitrocellulose forme les couches de nitrocellulose tandis que le matériau de réaction est laissé dans les couches de nitrocellulose en forme de poudre et la seconde zone étant configurée pour délivrer l'échantillon de fluide en absorbant l'échantillon de fluide qui vient de la première zone et en délivrant l'échantillon de fluide à la troisième zone et la troisième zone étant configurée pour faire réagir et détecter des analytes de l'échantillon de fluide, chaque zone, la seconde et la troisième zone ayant une largeur d'au moins 0,3 mm et la couche de nitrocellulose de la troisième zone étant capable d'absorber un volume constant d'échantillon de fluide venant de la seconde zone, la couche de nitrocellulose de la seconde zone ayant une épaisseur moyenne qui est inférieure à une épaisseur moyenne de la couche de nitrocellulose de la troisième zone.

2. Bandelette analytique selon la revendication 1 dans laquelle la solution de nitrocellulose est un mélange de poudre de nitrocellulose et d'un solvant organique qui contient des esters et des cétones.

3. Bandelette analytique selon la revendication 2 dans laquelle la poudre de nitrocellulose est mélangée au solvant qui contient des esters et des cétones dans un rapport volumétrique d'1 à 9.

4. Bandelette analytique selon la revendication 1 dans laquelle le substrat est fait en un matériau biocompatible.

5. Bandelette analytique selon la revendication 1 dans laquelle le canal comprend de plus une quatrième zone ayant un fond équipé d'une couche de nitrocellulose ayant une conformation de matrice creuse pour loger l'excédent d'échantillon de fluide.

6. Bandelette analytique selon la revendication 5 dans laquelle le matériau de réaction est formé en la conformation de matrice creuse de la couche de nitrocellulose de la quatrième zone, le matériau de réaction dans la conformation de matrice creuse étant sous forme de poudre et est formé en mélangeant une solution de réaction contenant le matériau de réaction avec la solution de nitrocellulose et en moulant ensuite sur les fonds de la seconde, de la troisième et de la quatrième zone, ceci étant suivi par un processus de séchage si bien que la solution de nitrocellulose forme les couches de nitrocellulose tandis que le matériau de réaction est laissé dans les couches de nitrocellulose sous forme de poudre.
